(19) Europäisches Patentamt
European Patent Office
Office européen des brevets

(11) EP 0 732 334 A1

(12) DEMANDE DE BREVET EUROPEEN

(43) Date de publication: **18.09.1996 Bulletin 1996/38**

(21) Numéro de dépôt: **96400452.7**

(22) Date de dépôt: **04.03.1996**

(51) Int Cl.[6]: **C07D 471/06**, C07D 498/06, A61K 31/535, A61K 31/435 // (C07D471/06, 235:00, 221:00), (C07D498/06, 265:00, 235:00)

(84) Etats contractants désignés:
**AT BE CH DE DK ES FI FR GB GR IE IT LI LU NL PT SE**

(30) Priorité: **13.03.1995 FR 9502863**

(71) Demandeur: **SYNTHELABO**
**F-92350 Le Plessis Robinson (FR)**

(72) Inventeurs:
- **Even, Luc**
**F-75013 Paris (FR)**
- **Jegham, Samir**
**F-95100 Argenteuil (FR)**
- **Defosse, Gérard**
**F-75013 Paris (FR)**
- **Aletru, Michel**
**F-75020 Paris (FR)**

(74) Mandataire: **Thouret-Lemaitre, Elisabeth et al**
**SYNTHELABO,**
**Service Brevets,**
**B.P. 72**
**92352 Le Plessis-Robinson Cédex (FR)**

(54) **Dérivés de pipéridine, leur procédé de préparation et leur application en thérapeutique**

(57) Dérivés de pipéridine de formule générale (I)

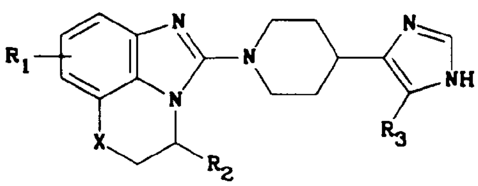

(I)

dans laquelle
X représente un atome d'oxygène ou un groupe méthylène,
$R_1$ représente un atome de chlore ou de fluor ou un groupe méthyle, méthoxy ou amino, et
$R_2$ et $R_3$ représentent indépendamment l'un de l'autre, des atomes d'hydrogène ou des groupes méthyle.
Application en thérapeutique.

EP 0 732 334 A1

## Description

La présente invention a pour objet des dérivés de pipéridine, leur procédé de préparation et leur application en thérapeutique.

Les composés de l'invention répondent à la formule générale (I)

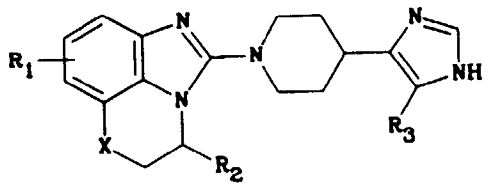

(I)

dans laquelle

X représente un atome d'oxygène ou un groupe méthylène,
$R_1$ représente un atome de chlore ou de fluor ou un groupe méthyle, méthoxy ou amino, et
$R_2$ et $R_3$ représentent, indépendamment l'un de l'autre, des atomes d'hydrogène ou des groupes méthyle.

Les composés préférés selon l'invention sont ceux pour lesquels,

X représente un atome d'oxygène et
$R_1$ est en position 8 sur le cycle.

Les composés de formule (I) particulièrement préférés sont la *(S)*-8-fluoro-4-méthyl-2-[4-(5-méthyl-1*H*-imidazol-4-yl) pipéridin-1-yl]-4,5-dihydroimidazo[1,5,4-de][1,4]benzoxazine et la (S)-8-fluoro-4-méthyl-2-[4-(1*H*-imidazol-4-yl) pipéridin-1-yl]-4,5-dihydroimidazo[1,5,4-de] [1,4] benzoxazine.

Les composés de l'invention peuvent se présenter sous forme de bases libres ou de sels d'addition à des acides pharmaceutiquement acceptables.

Certains composés de formule (I) comportent un atome de carbone asymétrique. Ils peuvent donc exister sous forme d'énantiomères. Ces énantiomères purs ou sous forme de mélanges, y compris de mélanges racémiques font partie de l'invention.

Les formes mésomères des composés de formule (I) font également partie de l'invention.

Les composés de formule (I) peuvent être préparés par réaction d'un composé de formule (II) dans laquelle X, $R_1$ et $R_2$ sont définis comme dans la formule (I) et Y représente un atome d'halogène, en particulier un atome de chlore, avec un composé de formule (III) dans laquelle $R_3$ est défini comme dans la formule (I), selon le schéma 1.

### Schéma 1

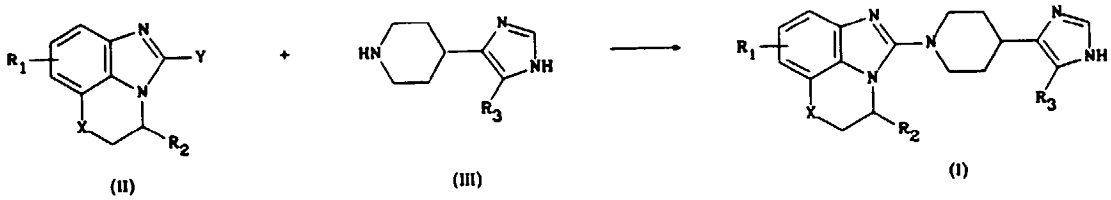

(II) (III) (I)

Les composés de départ sont disponibles dans le commerce ou décrits dans la littérature ou peuvent être préparés selon des méthodes qui y sont décrites ou qui sont connues de l'homme du métier.

Ainsi le composé de formule (III) dans laquelle $R_3$ est un atome d'hydrogène est décrit dans Arch. Pharmaz., 306 (12), 934-942 (1973).

Le composé de formule (III) dans laquelle $R_3$ est un groupe méthyle peut être préparé selon le procédé décrit dans la demande de brevet européen 0507650.

Les composés de formule (II) dans laquelle Y est un atome de chlore et X, $R_1$ et $R_2$ sont définis comme dans la formule (I), peuvent être préparés par réduction puis réaction avec l'urée, d'un composé de formule (IV)

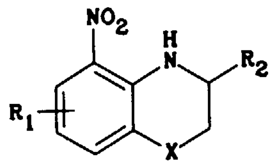

(IV)

puis traitement du composé de formule (V) ainsi obtenu

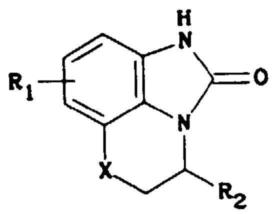

(V)

par le chlorure de phosphoryle.

Les composés de formule (IV) peuvent être préparés par réaction d'un composé de formule (VI)

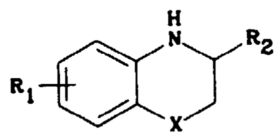

(VI)

avec le dicarbonate de bis(1,1-diméthyléthyle), nitration du composé de formule (VII)

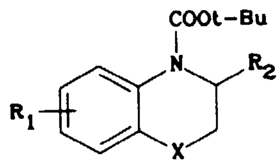

(VII)

et hydrolyse du composé de formule (VIII)

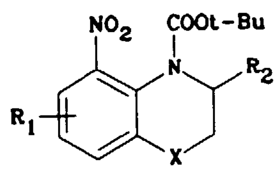

(VIII)

Certains composés de formule (IV) peuvent également être préparés à partir d'un composé de formule (IX)

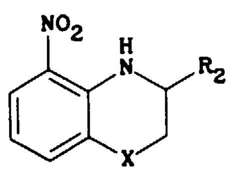

(IX)

dans laquelle X et $R_2$ sont définis comme dans la formule (I). En particulier les composés de formule (IVa)

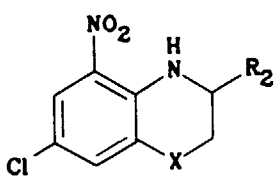

(IVa)

peuvent être préparés par chloration des composés de formule (IX).

La préparation du composé de formule (IX) dans laquelle $R_2$ est un groupe méthyle et X un atome d'oxygène est décrite dans la demande de brevet européen 0646583.

Le composé de formule (IX) dans laquelle $R_2$ est un atome d'hydrogène et X un atome d'oxygène peut être préparé

par un procédé analogue.
Les composés de formule (IVb)

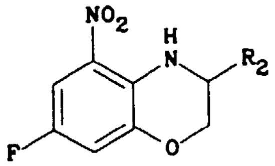

(IVb)

dans laquelle $R_2$ est tel que défini dans la formule (I) peuvent également être préparés par nitration d'un composé de formule (X)

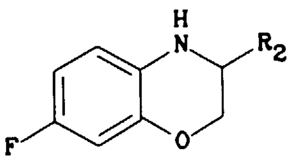

(X)

Les composés de formule (X) sont préparés par cyclisation du 2-bromo-5-fluorophénol avec un composé de formule (XI)

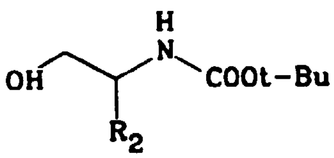

(XI)

Les exemples suivants illustrent la préparation des composés selon la présente invention. Les microanalyses et les spectres RMN ont confirmé la structure des produits obtenus. Les rapports (x:y) correspondent au rapport (acide: base). Les numéros entre parenthèses renvoient au tableau donné plus loin qui illustre les structures chimiques et les propriétés physiques de quelques composés selon l'invention.

Exemple 1 (composé no 1)

*(S)*-8-chloro-4-méthyl-2-[4-(5-méthyl-1*H*-imidazol-4-yl)pipéridin-1-yl]-4,5-dihydroimidazo[1,5,4-de][1,4] benzoxazine

1.1. (*S*)-7-chloro-3-méthyl-5-nitro-3,4-dihydro-2*H*-1,4-benzoxazine

On dissout 5,82 g (0,030 mole) de (S)-3-méthyl-5-nitro-3,4-dihydro-2*H*-1,4-benzoxazine dans 135 ml d'acide acétique, à 45 °C. On ajoute rapidement 5,16 g (0,038 mole) de *N*-chlorosuccinimide, on agite le milieu réactionnel pendant 4 heures à 50 °C puis on le verse dans de l'eau et on extrait 3 fois avec de l'éther diéthylique. On réunit les phases organiques, on les lave à l'eau puis avec une solution aqueuse d'hydroxyde de sodium, on les sèche et on évapore le solvant à sec. On obtient 6,7 g de produit.
Point de fusion : 95 °C

1.2. (*S*) -8-chloro-4-méthyl-4,5-dihydroimidazo[1,5,4-*de*] [1,4]benzoxazin-2(*1H*)-one

On introduit dans un appareil de Parr, une suspension de 8,9 g (0,039 mole) de (*S*)-7-chloro-3-méthyl-5-nitro-3,4-dihydro-2*H*-1,4-benzoxazine dans 250 ml d'éthanol et une quantité catalytique de nickel de Raney. On hydrogène pendant 2 heures à température ambiante sous une pression de 70 kPa 5 (10 psi). On filtre ensuite le catalyseur, on le lave à l'éthanol, on récupère le filtrat et on évapore le solvant à sec.
Au résidu ainsi obtenu, on ajoute 3,4 g (0,056 mole) d'urée et on chauffe au bain d'huile à 170-180 °C pendant 1 heure. On reprend ensuite le solide obtenu dans un mélange d'eau et d'éther diéthylique (50:50), on essore le précipité formé, on le lave à l'éther diéthylique puis à l'eau et on le sèche sous vide sur pentoxyde de phosphore. On obtient 6,3 g de produit que l'on purifie par chromatographie sur colonne de gel de silice avec un mélange de dichlorométhane et de méthanol (97:3).
On obtient 5,2 g de produit.

1.3. (*S*)-2,8-dichloro-4-méthyl-4,5-dihydroimidazo[1,5,4-*de*] [1,4]benzoxazine

On verse 100 ml de chlorure de phosphoryle sur 5,2 g (0,023 mole) de (*S*)-8-chloro-4-méthyl-4,5-dihydroimidazo [1,5,4-de][1,4]benzoxazin-2(1*H*)-one. On chauffe le mélange au reflux pendant 2 heures puis on évapore le solvant à sec et on reprend le résidu par de l'eau glacée puis par une solution d'ammoniaque concentrée. On extrait ensuite avec de l'éther diéthylique, on réunit les phases organiques, on les sèche et on évapore le solvant à sec. On obtient 4,5 g de résidu que l'on purifie par chromatographie sur colonne de gel de silice avec un mélange d'hexane et d'acétate d'éthyle (97:3).
On obtient 4,1 g de produit sous forme d'huile.

1.4. (*S*)-8-chloro-4-méthyl-2-[4-(5-méthyl-1*H*-imidazol-4-yl)pipéridin-1-yl]-4,5-dihydroimidazo[1,5,4-*de*][1,4] benzoxazine

On chauffe à 120 °C pendant 12 heures sous agitation, un mélange de 1,35 g (0,0082 mole) de 4-(5-méthyl-1*H*-imidazol-4-yl)pipéridine, 1 g (0,0041 mole) de (*S*)-2,8-dichloro-4-méthyl-4,5-dihydroimidazo[1,5,4-*de*][1,4]benzoxazine et 4,5 ml d'alcool isoamylique. On évapore ensuite le solvant à sec et on purifie le résidu par chromatographie sur colonne de gel de silice avec un mélange de dichlorométhane, de méthanol et d'ammoniaque (95:5:0,5).
Après recristallisation dans l'acétone, on obtient 0,7 g de produit sous forme de base.
Point de fusion : 208 °C
$[\alpha]_D^{20}$ = - 23,8 ° (c = 0,01 ; méthanol)

<u>Exemple 2</u> (composé no 2)

(*S*)-8-chloro-2-[4-(1*H*-imidazol-4-yl)pipéridin-1-yl]-4-méthyl-4,5-dihydroimidazo[1,5,4-*de*][1,4]benzoxazine

A partir d'un mélange de (*S*)-2,8-dichloro-4-méthyl-4,5-di hydroimidazo[1,5,4-*de*][1,4]benzoxazine et de 4-(1*H*-imidazol-4-yl)pipéridine, traité dans les conditions de l'exemple 1.4, on obtient, après recristallisation dans l'acétone, 0,6 g de produit sous forme de base.
Point de fusion : 215 °C
$[\alpha]_D^{20}$ = - 3,7 ° (c = 0,01 ; méthanol)

<u>Exemple 3</u> (composé no 5)

8-fluoro-2-[4-(1*H*-imidazol-4-yl)pipéridin-1-yl]-4-méthyl-5,6-dihydro-4*H*-imidazo[4,5,1-*ij*]quinoléine

3.1. 6-fluoro-2-méthy1-1,2,3,4-tétrahydroquino1éine-1-carboxylate de 1,1-diméthyléthyle

On chauffe à 60 °C pendant 3 jours, un mélange de 16,5 g (0,1 mole) de 6-fluoro-2-méthyl-1,2,3,4-tétrahydroquinoléine et 32 g (0,146 mole) de dicarbonate de bis(1,1-diméthyléthyle) dans 100 ml de tétrahydrofurane. On évapore ensuite le solvant à sec et on chasse à la pompe à palette le restant de dicarbonate. On purifie le résidu par chromatographie sur gel de silice avec un mélange d'hexane et d'acétate d'éthyle (90:10).
On obtient 24,5 g de produit sous forme d'huile.

3.2. 6-fluoro-2-méthyl-8-nitro-1,2,3,4-tétrahydroquinoléine-1-carboxylate de 1,1-diméthyléthyle

On refroidit à - 78 °C une solution de 18,4 g (0,0697 mole) de 6-fluoro-2-méthyl-1,2,3,4-tétrahydroquino1éine-1-carboxylate de 1,1-diméthyléthyle et 13,8 ml de *N,N,N',N'*-tétraméthyléthylènediamine anhydre dans 350 ml d'éther diéthylique anhydre. On ajoute ensuite en 30 minutes, 64,2 ml d'une solution 1,3 N d'iodure de *sec*-butyle dans un mélange de cyclohexane et de pentane. On agite pendant 1 heure à - 78 °C puis on ajoute 12,3 g (0,103 mole) de nitrate d'isobutyle. On agite le milieu réactionnel pendant 1,5 heures à - 70°C, puis on le neutralise en le versant dans de l'eau glacée. On extrait avec de l'éther diéthylique, on réunit les phases organiques, on les lave à l'eau, on les sèche, puis on évapore le solvant à sec. On purifie le résidu par chromatographie sur colonne de gel de silice en éluant par un mélange d'hexane et d'acétate d'éthyle (90:10). On obtient 8,1 g de produit.
3.3. 6-fluoro-2-méthyl-8-nitro-1,2,3,4-tétrahydroquinoléine On chauffe à la température de reflux pendant une journée, un mélange de 8,6 g (0,0261 mole) de 6-fluoro-2-méthyl-8-nitro-1,2,3,4-tétrahydroquinoléine-1-carboxylate de 1,1-diméthyléthyle, 30 ml d'acide chlorhydrique concentré, 20 ml de toluène et 30 ml d'eau. On laisse ensuite revenir à la température ambiante, on décante et on extrait au toluène. On réunit les phases organiques, on les lave à l'eau, on les sèche, puis on évapore le solvant à sec.

On obtient 5,6 g de produit.
Point de fusion : 56 °C

3.4. 8-fluoro-4-méthyl-5,6-dihydro-4*H*-imidazo[4,5, 1-*ij*] quinoléine-2(1*H*)-one

On introduit dans un appareil de Parr, une suspension de 5,5 g (0,026 mole) de 6-fluoro-2-méthyl-8-nitro-1,2,3,4-tétrahydroquinoléine dans 150 ml d'éthanol et une quantité catalytique de nickel de Raney puis on hydrogène pendant 1 heure à température ambiante sous une pression de 0,21 MPa (30 psi). On filtre ensuite le catalyseur, on le lave avec de l'éthanol, on récupère le filtrat et on évapore le solvant à sec. On ajoute le résidu à 2,6 g (0,043 mole) d'urée et on chauffe au bain d'huile à 175 °C pendant 1,5 heures. On reprend le solide obtenu par un mélange d'eau et d'éther diéthylique (50:50), puis on essore le précipité formé, on le lave à l'éther diéthylique et à l'eau et on le sèche sous vide sur pentoxyde de phosphore.
On obtient 4,2 g de produit.

3.5. 2-chloro-8-fluoro-4-méthyl-5,6-dihydro-4*H*-imidazo [4,5,1-*ij*]quinoléine

On verse 100 ml de chlorure de phosphoryle sur 4,2 g (0,02 mole) de 8-fluoro-4-méthyl-5,6-dihydro-4*H*-imidazo [4,5,1-*ij*]quinoléine-2(1*H*)-one puis on chauffe le mélange au reflux pendant 2 heures. On évapore ensuite le solvant à sec et on reprend le résidu par de l'eau glacée puis par une solution d'ammoniaque concentrée. On extrait avec de l'éther diéthylique, on réunit les phases organiques, on les sèche, puis on évapore le solvant à sec. On purifie le résidu par chromatographie sur colonne de gel de silice en éluant avec un mélange d'hexane et d'acétate d'éthyle (70:30).
On obtient 2,4 g de produit.
Point de fusion : 80 °C

3.6. 8-fluoro-2-[4-(1*H*-imidazol-4-yl)pipéridin-1-yl]-4-méthyl-5,6-dihydro-4*H*-imidazo[4,5,1-*ij*]quinoléine

On chauffe à 120 °C pendant 18 heures sous agitation, un mélange de 0,94 g (0,00625 mole) de 4-(1*H*-imidazol-4-yl) pipéridine, 0,7 g (0,0031 mole) de 2-chloro-8-fluoro-4-méthyl-5,6-dihydro-4H-imidazo[4,5,1-*ij*]quinoléine et 3,5 ml d'alcool isoamylique. On évapore ensuite le solvant à sec et on purifie le résidu par chromatographie sur colonne de gel de silice avec un mélange de dichlorométhane, de méthanol et d'ammoniaque (95:5:0,5).
Après recristallisation dans l'acétone, on obtient 0,85 g de produit sous forme de base.
Point de fusion : 175-177 °C

<u>Exemple 4</u> (composé no 6)

8-fluoro-4-méthyl-2-[4-(5-méthyl-1*H*-imidazol-4-yl)pipéridin-1-yl]-5,6-dihydro-4*H*-imidazo[4,5,1-*ij*]quinoléine

A partir de 0,7 g (0,0031 mole) de 2-chloro-8-fluoro-4-méthyl-5,6-dihydro-4*H*-imidazo[4,5,1-*ij*]quinoléine et de 1 g (0,00623 mole) de 4-(5-méthyl-1*H*-imidazol-4-yl)pipéridine, traités dans les conditions de l'exemple 3.6, on obtient après recristallisation dans l'acétone, 0,7 g de produit sous forme de base.
Point de fusion : 255 °C

<u>Exemple 5</u> (composé no 8)

(*S*)-4-méthyl-2-[4-(5-méthyl-1*H*-imidazol-4-yl) pipéridin-1-yl]-4,5-dihydroimidazo[1,5,4-*de*][1,4]benzoxazin-8-amine

5.1. (*S*)-5,7-dinitro-3-méthyl-3,4-dihydro-2*H*-1,4-benzoxazine On dissout 7,7 g (0,04 mole) de (*S*)-3-méthyl-5-nitro-3,4-dihydro-2*H*-1,4-benzoxazine dans 176 ml d'acide acétique, à 60 °C. On additionne goutte à goutte 2,8 ml (0,044 mole) d'acide nitrique, on agite le milieu réactionnel pendant 45 minutes à 60 °C puis on ajoute 176 ml d'eau. On refroidit à 0 °C et on essore le précipité formé, on le lave à l'eau et on le sèche sous vide.
On obtient 8,13 g de produit.
Point de fusion : 170 °C

5.2. (*S*)-3-méthyl-7-nitro-3,4-dihydro-2*H*-1,4-benzoxazin-5-amine

On chauffe à 50 °C, 7,63 g (0,032 mole) de (*S*)-5,7-dinitro-3-méthyl-3,4-dihydro-2*H*-1,4-benzoxazine et on additionne goutte à goutte une solution de 30,52 g (0,391 mole) de sulfure de sodium et de 10,70 g d'hydrogénocarbonate de sodium dissous dans 46 ml d'eau.

On porte lentement ce mélange réactionnel à la température de reflux, puis après 30 minutes d'agitation on verse un volume d'eau. On laisse refroidir et on extrait trois fois avec de l'éther éthylique. On réunit les phases organiques, on les lave à l'eau, on les sèche puis on évapore le solvant à sec. On obtient 5,98 g de produit.

5.3. (*S*)-4-méthyl-8-nitro-4,5-dihydroimidazo[1,5,4-*de*][1,4] benzoxazin-2(1*H*)-one

A 5,98 g (0,029 mole) de (*S*)-3-méthyl-7-nitro-3,4-dihydro-2*H*-1,4-benzoxazin-5-amine, on ajoute 3,43 g (0,057 mole) d'urée et on chauffe le mélange pendant 2 heures à 175 °C. On reprend par de l'eau bouillante, on filtre le précipité et on essore avec de l'eau. On extrait le filtrat avec du dichlorométhane, on lave avec de l'eau, on sèche et on évapore sous vide. On purifie le résidu par chromatographie sur colonne de gel de silice en éluant par un mélange dichlorométhane:méthanol (98:2).
On obtient 3,86 g de produit.

5.4. (*S*)-2-chloro-4-méthyl-8-nitro-4,5-dihydroimidazo [1,5,4-*de*][1,4]benzoxazine

On chauffe à la température de reflux pendant trois heures, 3,86 g (0,021 mole) de *(S)*-4-méthyl-8-nitro-4,5-dihydroimidazo[1,5,4-*de*][1,4]benzoxazin-2(1*H*)-one en présence de 76 ml de chlorure de phosphoryle. On évapore le solvant à sec et on reprend le résidu par de la glace. On alcalinise le milieu réactionnel avec de l'ammoniaque aqueux, on filtre le précipité formé, on le rince avec de l'eau et on l'essore. On le sèche dans une étuve à vide. On purifie le résidu par chromatographie sur colonne de gel de silice en éluant par un mélange dichlorométhane:méthanol (99:1).
On obtient 3,6 g de produit.

5.5. (*S*)-4-méthyl-8-nitro-2-[4-(5-méthyl-1*H*-imidazol-4-yl) pipéridin-1-yl]-4,5-dihydroimidazo[1,5,4-*de*][1,4] benzoxazine

On chauffe à 120 °C pendant 5 heures sous agitation, un mélange de 1,87 g (0,00788 mole) de 4-(5-méthyl-1*H*-imidazol-4-yl)pipéridine, 1 g (0,00394 mole) de *(S)*-2-chloro-4-méthyl-8-nitro-4,5-dihydroimidazo[1,5,4-*de*][1,4]benzoxazine et 5 ml d'alcool isoamylique. On évapore le solvant à sec et on purifie le résidu par chromatographie sur colonne de gel de silice en éluant avec un mélange de dichlorométhane, de méthanol et d'ammoniaque (94:6:0,6).
Après recristallisation dans l'éthanol, on obtient 1,3 g de produit sous forme de base.
Point de fusion : 125 °C

5 5.6 (*S*)-4-méthyl-2-[4-(5-méthyl-1*H*-imidazol-4-yl) pipéridin-1-yl]-4,5-dihydroimidazo[1,5,4-*de*][1,4] benzoxazin-8-amine

On introduit dans une fiole de Parr, une suspension de 1,1 g (0,00287 mole) de (*S*)-4-méthyl-8-nitro-2-[4-(5-méthyl-1*H*- imidazol-4-yl)pipéridin-1-yl]-4,5-dihydroimidazo[1,5,4-*de*] [1,4]benzoxazine dans 50 ml d'éthanol et une quantité catalytique d'oxyde de platine puis on hydrogène pendant 1 heure à température ambiante sous une pression de 0,28 MPa (40 psi). On filtre le catalyseur, on le lave à l'éthanol et à l'acétate d'éthyle, on récupère le filtrat et on évapore le solvant à sec.
On obtient 0,8 g de produit qui cristallise sous forme de base.
Point de fusion : 245 °C
$[\alpha]_D^{20}$ = -23,4 ° (c = 0,01 ; méthanol).

<u>Exemple 6</u> (composé no 7)

(*S*)-2-[4-(1*H*-imidazol-4-yl)pipéridin-1-yl]-4-méthyl-4,5-dihydroimidazo[1,5,4-*de*][1,4]benzoxazin-8-amine

A partir d'un mélange de (*S*)-2-chloro-4-méthyl-8-nitro-4,5-dihydroimidazo[1,5,4-*de*][1,4]benzoxazine et de 4-(1*H*-imidazol-4-yl)pipéridine, traité dans les conditions de l'exemple 5.5, on obtient après chromatographie sur colonne de gel de silice en éluant avec un mélange de dichlorométhane, de méthanol et d'ammoniaque (94:6:0,6), 1,3 g de produit que l'on traite dans les conditions de l'exemple 5.6.
On obtient 0,7 g de produit sous forme de base.
Point de fusion : 235-238 °C
$[\alpha]_D^{20}$ = - 1,4 ° (c = 0,01 ; méthanol)

Exemple 7 (composé no 14)

(*S*)-8-fluoro-4-méthyl-2-[4-(5-méthyl-1*H*-imidazol-4-yl) pipéridin-1-yl]-4,5-dihydroimidazo[1,5,4-*de*][1,4]benzoxazine

7.1. (*S*)-[2-(2-bromo-5-fluorophénoxy)-1-méthyléthyl] carbamate de 1,1-diméthyléthyle

Dans un ballon tricol de 4 litres contenant 2,3 litres de toluène, on place 130 g (0,74 mole) de (*S*)-(2-hydroxy-1-méthyléthyl)carbamate de 1,1-diméthyléthyle et 191 g (0,728 mole) de triphénylphosphine. On refroidit le mélange au bain de glace et on ajoute goutte à goutte 115 ml (0,726 mole) de diéthylazodicarboxylate. On laisse sous agitation pendant 1 heure, on ajoute goutte à goutte 100 g (0,52 mole) de 2-bromo-5-fluorophénol, on agite pendant 2 heures à 0 °C, on laisse la température du milieu réactionnel revenir à la température ambiante et on laisse sous agitation pendant une nuit. Ensuite on filtre le précipité obtenu, on lave le filtrat avec de la soude 1 N et de l'eau, on sèche et on évapore. On purifie le résidu obtenu par chromatographie sur colonne de gel de silice en éluant avec un mélange de dichlorométhane et d'heptane (70:30).
On obtient 170 g de produit.
$[\alpha]_D^{20}$ = - 45,6 ° (c= 0,01 ; dichlorométhane)

7.2. (*S*)-1-(2-bromo-5-fluorophénoxy)propan-2-amine

On chauffe au reflux pendant 2 heures, 170 g (0,48 mole) de (*S*)-[2-(2-bromo-5-fluorophénoxy)-1-méthyléthyl] carbamate de 1,1-diméthyléthyle dissous dans 520 ml d'eau et 260 ml d'acide chlorhydrique 12 N. On verse 500 ml d'eau glacée et on extrait une fois avec 600 ml de toluène et une fois avec 400 ml d'éther. On refroidit la phase aqueuse dans un bain d'eau glacée et on alcalinise avec de la soude 10 N. On extrait trois fois avec 500 ml d'éther, on réunit les phases organiques, on les lave deux fois à l'eau et une fois avec une solution aqueuse saturée en chlorure de sodium, on sèche et on évapore sous pression réduite.
On obtient 118 g de produit sous forme d'huile.
$[\alpha]_D^{20}$ = + 2,8 ° (c = 0,01 ; dichlorométhane)

7.3. (*S*)-7-f1uoro-3-méthyl-3,4-dihydro-2*H*-1,4-benzoxazine

Dans un tricol de 4 litres, sous atmosphère inerte, on place 72 g (0,768 mole) de tert-butylate de sodium dans 1 litre de toluène. On ajoute 5 g de palladium tétrakis(triphénylphosphine), puis une solution de 120 g (0,48 mole) de *(S)*-1-(2-bromo-5-fluorophénoxy)propan-2-amine dans 250 ml de toluène. On chauffe le milieu réactionnel à 110 °C et on laisse agiter pendant 2,5 heures. On ajoute de nouveau 3 g de catalyseur et après 5,5 heures d'agitation à 110 °C, on laisse refroidir le milieu réactionnel et on ajoute 1 litre d'eau. On agite, on laisse décanter et on extrait avec du toluène. On réunit les toluène. On réunit les phases organiques, on les lave à l'eau, on les sèche et on évapore le solvant sous pression réduite. On purifie par chromatographie sur colonne de gel de silice en éluant par du dichlorométhane.
On obtient 21,5 g de produit.
$[\alpha]_D^{20}$ = - 4,8 ° (c = 0,01 ; méthanol)

7.4. (*S*)-4-acétyl-7-fluoro-3-méthyl-3,4-dihydro-2*H*-1,4-benzoxazine

On refroidit au bain de glace une solution de 21,5 g (0,128 mole) de (*S*)-7-f1uoro-3-méthyl-3,4-dihydro-2*H*-1,4-benzoxazine dans 60 ml de pyridine, on ajoute 15,1 g (0,15 mole) d'anhydride acétique, on laisse revenir à température ambiante et on agite le mélange pendant 48 heures. On verse sur de l'eau glacée et on extrait deux fois à l'éther. On réunit les phases organiques, on les lave successivement à l'eau, à l'acide chlorhydrique dilué, à l'eau et à la saumure, puis on les sèche et on évapore le solvant sous pression réduite.
On obtient 24,2 g de produit.
$[\alpha]_D^{20}$ = + 102,9 ° (c = 0,01 ; dichorométhane)

7.5. (*S*)-7-fluoro-3-méthyl-5-nitro-3,4-dihydro-2*H*-1,4-benzoxazine

On dissout 24 g (0,114 mole) de (*S*)-4-acétyl-7-fluoro-3-méthyl-3,4-dihydro-2*H*-1,4-benzoxazine dans 350 ml d'acide acétique. On additionne goutte à goutte 8,4 ml (0,134 mole) d'acide nitrique et on laisse le milieu réactionnel sous agitation pendant 4 heures, à environ 100 °C. On évapore l'acide acétique sous pression réduite, on ajoute 100 ml de toluène puis 500 ml de soude 3 N et on laisse agiter à la température de reflux pendant 3 heures. On décante le milieu réactionnel et on extrait au toluène. On réunit les phases organiques, on les lave à l'eau, on les sèche et on évapore

5 sous pression réduite. On purifie le résidu obtenu par chromatographie sur colonne de gel de silice en éluant avec un mélange de dichlorométhane et d'heptane (50:50). On sépare deux produits.
On obtient 3,8 g de (*S*)-7-fluoro-3-méthyl-5-nitro-3,4- dihydro-2*H*-1,4-benzoxazine
Point de fusion : 116 °C
$[\alpha]_D^{20}$ = - 65 ° (c = 0,016 ; dichlorométhane)
Et on obtient 15 g de son régioisomère, le (*S*)-7-fluoro-3-méthyl-6-nitro-3,4-dihydro-2*H*-1,4-benzoxazine
Point de fusion : 132 °C
$[\alpha]_D^{20}$ = -79,7 ° (c = 0,01 ; dichlorométhane)

7.6. (*S*)-7-fluoro-3-méthyl-3,4-dihydro-2*H*-1,4-benzoxazin-5-amine

On introduit dans une fiole de Parr, une solution de 3,45 g (0,0162 mole) de (*S*)-7-fluoro-3-méthyl-5-nitro-3,4-dihydro-2*H*-1,4-benzoxazine dans 90 ml d'éthanol et une quantité catalytique d'oxyde de platine. On hydrogène pendant 1 heure à température ambiante sous une pression de 0,28 MPa (40 psi). On filtre le catalyseur, on le lave avec de l'éthanol, on récupère le filtrat et on évapore le solvant à sec. On obtient 2,9 g de produit cristallisé.
Point de fusion : 72 °c
$[\alpha]_D^{20}$ = - 70,3 ° (c = 0,01; dichlorométhane)

7.7. (*S*)-8-fluoro-4-méthyl-4,5-dihydroimidazo[1,5,4-*de*] [1,4]benzoxazine-2(1*H*)-one

On chauffe à 175 °C au bain d'huile pendant 1,5 heures, 2,9 g (0,0162 mole) de (*S*)-7-fluoro-3-méthyl-3,4-dihydro-2*H*-1,4-benzoxazin-5-amine en présence de 1,8 g (0,03 mole) d'urée. On reprend le résidu par un mélange eau:éther (50:50), on triture pour obtenir un précipité que l'on filtre, on lave à l'eau et à l'éther puis on sèche.
On obtient 3,1 g de produit.
Point de fusion : 175 °C
$[\alpha]_D^{20}$ = + 33,9 ° (c = 0,01 ; diméthylformamide)

7.8. *(S)*-2-chloro-8-fluoro-4-méthyl-4,5-dihydroimidazo [1,5,4-*de*][1,4]benzoxazine

On chauffe à la température de reflux pendant 3 heures, 3,1 g (0,0148 mole) de (S)-8-fluoro-4-méthyl-4,5-dihydroimidazo [1,5,4-*de*][1,4]benzoxazine-2(1*H*)-one dans 70 ml de chlorure de phosphoryle. On évapore le solvant à sec, on reprend le résidu par de l'eau glacée et on ajuste le pH du milieu à 8 avec de l'ammoniaque. On extrait deux fois à l'éther, on réunit les phases organiques, on les lave à l'eau, on les sèche et on évapore le solvant à sec. On purifie par chromatographie sur colonne de gel de silice en éluant avec un mélange d'acétate d'éthyle et d'heptane (50:50). On obtient 1,85 g de produit.
Point de fusion : 102 °C
$[\alpha]_D^{20}$ = - 15,85 °(c = 0,01; dichlorométhane)

7.9. (*S*)-8-fluoro-4-méthyl-2-[4-(5-méthyl-1*H*-imidazol-4-yl) pipéridin-1-yl]-4,5-dihydroimidazo[1,5,4-*de*][1,4] benzoxazine

On chauffe à 120 °C pendant 24 heures sous agitation, un mélange de 1,47 g (0,00617 mole) de 4-(5-méthyl-1*H*-imidazol-4-yl)pipéridine, 0,7 g (0,00308 mole) de (*S*)-2-chloro-8-fluoro-4-méthyl-4,5-dihydroimidazo[1,5,4-*de*][1,4] benzoxazine et 4 ml d'alcool isoamylique. On évapore le solvant à sec et on purifie le résidu par chromatographie sur colonne de gel de silice en éluant par un mélange de dichlorométhane, de méthanol et d'ammoniaque (95/5/0,5).
Après recristallisation dans l'éthanol, on obtient 0,6 g de produit sous forme de base.
Point de fusion : 224 °C
$[\alpha]_D^{20}$ = - 11,0 ° (c = 0,991 ; méthanol)

<u>Exemple 8</u> (composé no 13)

*(Z)*-but-2-ènedioate de (*S*)-8-fluoro-2-[4-(1*H*-imidazol-4-yl)pipéridin-1-y1]-4-méthy1-4,5-dihydroimidazo[1,5,4-*de*] [1,4]benzoxazine (2:1)

A partir d'un mélange de (*S*)-2-chloro-8-fluoro-4-méthyl-4,5-dihydroimidazo[1,5,4-*de*][1,4]benzoxazine et de 4-(1*H*-imidazol-4-yl)pipéridine, traité dans les conditions de l'exemple 5 7.9., on obtient le produit sous forme de base que l'on purifie par chromatographie sur colonne de gel de silice en éluant avec un mélange de dichlorométhane, de méthanol et d'ammoniaque (95/5/0,5).

En ajoutant à la base deux équivalents d'acide maléique dans du méthanol, on obtient 0,26 g de maléate.
Point de fusion : 142 °C
$[\alpha]_D^{20}$ = + 8,13 °(c = 0,504 ; méthanol)

Légende du tableau :

*dans la colonne "Sel" :* le rapport entre parenthèses représente le rapport (sel:base) ; l'absence de toute mention signifie que le produit est sous forme de base ; "HCl" correspond à un chlorhydrate et "mal." correspond à un maléate

**Tableau**

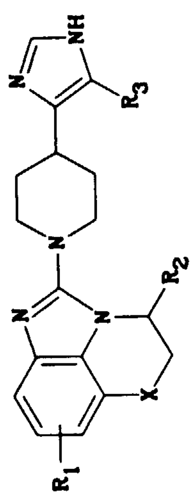

(I)

| N° | X | $R_1$ | $R_2$ | $R_3$ | Sel | F (°C) | $[\alpha]_D^{20}$ (c=0,01; méthanol) |
|---|---|---|---|---|---|---|---|
| 1 | O | 8-Cl | (*S*)-$CH_3$ | -$CH_3$ | - | 208 | - 23,8 |
| 2 | O | 8-Cl | (*S*)-$CH_3$ | -H | - | 215 | - 3,7 |
| 3 | O | 8-Cl | -H | -H | HCl | 275 | - |
| 4 | O | 8-Cl | -H | -$CH_3$ | HCl | 234 | - |
| 5 | $CH_2$ | 8-F | -$CH_3$ | -H | - | 175-177 | - |
| 6 | $CH_2$ | 8-F | -$CH_3$ | -$CH_3$ | - | 255 | - |
| 7 | O | 8-$NH_2$ | (*S*)-$CH_3$ | -H | - | 235-238 | - 1,4 |
| 8 | O | 8-$NH_2$ | (*S*)-$CH_3$ | -$CH_3$ | - | 245 | - 23,4 |

| N° | X | $R_1$ | $R_2$ | $R_3$ | Sel | F (°C) | $[\alpha]_D^{20}$ (c=0,01; méthanol) |
|---|---|---|---|---|---|---|---|
| 9 | O | 8-F | -H | -H | - | 187 | - |
| 10 | O | 8-F | -H | $-CH_3$ | - | 210-217 | - |
| 11 | O | 8-F | $-CH_3$ | -H | - | 174 | - |
| 12 | O | 8-F | $-CH_3$ | $-CH_3$ | - | 242 | - |
| 13 | O | 8-F | (*S*)$-CH_3$ | -H | mal. | + 8,13[a] | 
| 14 | O | 8-F | (*S*)$-CH_3$ | $-CH_3$ | - | 224 | - 11[b] |
| 15 | O | 8-F | (*R*)$-CH_3$ | $-CH_3$ | - | 224 | + 11,1 |
| 16 | O | 8-F | (*R*)$-CH_3$ | -H | mal. | 142 | - 8,7[a] |

a : c = 0,504 ; méthanol.
b : c = 0,991 ; méthanol.

Les composés de l'invention ont fait l'objet d'essais pharmacologiques qui ont montré leur intérêt comme substances actives en thérapeutique.

Ils ont en particulier été testés quant à leurs effets inhibiteurs de la liaison du $[^3H]$-(*S*)-zacopride avec les récepteurs sérotoninergiques de type $5\text{-}HT_3$ du cortex de rat, selon la méthode décrite par N.M. Barnes et Coll., dans J. Pharm. Pharmacol., **40**, 548-551 (1988).

Des rats mâles Sprague-Dawley (OFA, Iffa credo) de 200 à 250 g sont euthanasiés et leur cerveau est prélevé. On dissèque le cortex et on l'homogénéise à l'aide d'un broyeur Polytron® (position 7,20 s) dans 20 volumes de tampon

Tris 25 mM (pH = 7,4, 22°C). On centrifuge l'homogénat pendant 10 mn à 45000xg (dans une centrifugeuse SORVALL munie d'un rotor SS34), puis le culot est remis en suspension dans 10 volumes de tampon Tris et incubé à 37°C pendant 10 mn sous agitation. On dilue ensuite la suspension à 20 volumes à l'aide de tampon Tris et on centrifuge dans les mêmes conditions que précédemment. Le culot obtenu est remis en suspension dans 5 volumes de tampon Tris puis réparti en fractions aliquotes de 5 ml qui sont congelées à - 80°C. Le jour de l'expérience, la préparation est décongelée à 4°C puis diluée 1,2 fois à l'aide du tampon d'incubation Tris-NaCl (Tris 25 mM, NaCl 150 mM, pH = 7,4, 22°C).

La suspension membranaire (100 µl, 1 mg de protéines) est incubée à 25°C pendant 25 mn en présence de 0,5 nM de $[^3H]$-(*S*)-zacopride (activité spécifique : 75-85 Ci/mmole, Amersham, Little Chalfont, Royaume Uni) dans un volume final de 500 µl de tampon Tris-NaCl, en l'absence ou en présence du composé à tester.

On arrête l'incubation par filtration en utilisant des filtres Whatman GF/B préalablement traités avec de la polyéthylènimine (0,1 %). Chaque tube réactionnel est prédilué avec 4 ml de tampon Tris-NaCl puis rincé 3 fois avec 4,5 ml de tampon Tris-NaCl.

Les filtres sont prédécoupés avant séchage dans l'étuve (120°C, 5mn). La radioactivité retenue sur les filtres est mesurée par scintigraphie liquide. La liaison non spécifique est déterminée en présence de 10 µM de MDL 72222.

Pour chaque concentration de composé étudié, on détermine le pourcentage d'inhibition de la liaison spécifique du $[^3H]$-(*S*)-zacopride, puis la concentration du composé inhibant 50 % de la liaison spécifique du $[^3H]$-(*S*)-zacopride ($CI_{50}$).

Les $CI_{50}$ des composés de l'invention se situent entre 0,5 nM et 1 µM.

Les composés de l'invention ont également été étudiés quant à leur affinité vis-à-vis des récepteurs 5-$HT_4$ dans le striatum de cobaye, selon la méthode décrite par Grossman et coll., dans Br. J. Pharmacol., **109**, 618-624 (1993).

On euthanasie des cobayes (Hartley, Charles River) de 300 à 400 g et on prélève leur cerveau. On excise les striata et on les congèle à - 80°C. Le jour de l'expérience, on décongèle le tissu à + 4°C dans 33 volumes de tampon Hépès-NaOH 50 mM (pH = 7,4 à 20°C) et on l'homogénéise à l'aide d'un broyeur Polytron®. On centrifuge l'homogénat pendant 10 mn à 48000xg, on récupère le culot, on le remet en suspension et on le centrifuge de nouveau dans les mêmes conditions. On suspend le culot final dans du tampon Hépès-NaOH (30 mg de tissu frais/ml). Cette suspension membranaire est utilisée telle quelle.

On incube 100 µl de la suspension membranaire à 0°C pendant 120 minutes, en présence de 0,1 nM de $[^3H]$GR113808 (activité spécifique : 80-85 Ci/mmole), dans un volume final de 1 ml de tampon Hépès-NaOH (50 mM, pH = 7,4), en l'absence ou en présence du composé à tester. On arrête l'incubation par filtration sur filtres Whatman GF/B®, préalablement traités avec de la polyéthylèneimine 0,1 %, on rince chaque tube par 4 ml de tampon à 0°C et on filtre de nouveau. On mesure la radioactivité retenue sur les filtres, par scintigraphie liquide. On détermine la liaison non spécifique en présence de sérotonine 30 µM.

La liaison spécifique représente 90 % de la radioactivité totale récupérée sur le filtre.

Pour chaque concentration de composé étudié, on détermine le pourcentage d'inhibition de la liaison spécifique du $[^3H]$GR113808 puis la concentration du composé testé qui inhibe 50 % de la liaison spécifique ($CI_{50}$).

Les $CI_{50}$ des composés de l'invention se situent entre 0,02 et 2 µM.

Les résultats des tests biologiques montrent que les composés de l'invention sont des antagonistes des récepteurs sérotoninergiques 5-$HT_3$ et/ou 5-$HT_4$.

Ils peuvent donc être utilisés pour le traitement et la prévention des désordres dans lesquels les récepteurs 5-$HT_3$ et 5-$HT_4$ sont impliqués, tels que nausées et vomissements, par exemple consécutifs à un traitement antitumoral ou à l'administration d'un anesthésique ; troubles du système nerveux central tels que la schizophrénie, la manie, l'anxiété et la dépression; troubles de la cognition tels que la démence sénile ou présénile d'Alzheimer ; dyskinésie, douleurs, migraines et maux de tête ; troubles de la dépendance ou du sevrage de l'alcool ou de drogues ; troubles de la fonction gastrointestinale tels que dyspepsie, ulcère peptique, aigreurs d'estomac, flatulences ; troubles du système cardiovasculaire et troubles respiratoires.

Ils peuvent également être utilisés pour le traitement et la prévention des désordres tels que diarrhée, colon irritable, reflux oesophagien, troubles moteurs intestinaux, troubles de la sécrétion intestinale, fibrose kystique du pancréas, syndrome carcinoïde et incontinence.

Les composés de l'invention, en association avec des excipients appropriés, peuvent être présentés sous toutes formes convenant à une administration orale ou parentérale, telles que comprimés, dragées, gélules, capsules, suspensions ou solutions buvables ou injectables, et dosées pour permettre une administration de 0,005 à 5 mg/kg, 1 à 4 fois par jour.

## Revendications

**1.** Dérivés de pipéridine de formule générale (I)

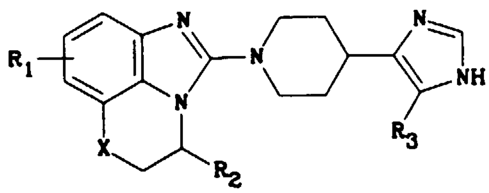

(I)

dans laquelle

X représente un atome d'oxygène ou un groupe méthylène,
$R_1$ représente un atome de chlore ou de fluor ou un groupe méthyle, méthoxy ou amino, et
$R_2$ et $R_3$ représentent indépendamment l'un de l'autre, des atomes d'hydrogène ou des groupes méthyle, sous forme d'énantiomères purs ou de mélanges d'énantiomères,
y compris de mélanges racémiques, ainsi que leurs sels d'addition à des acides pharmaceutiquement acceptables.

**2.** Dérivés selon la revendication 1 caractérisés en ce que

X représente un atome d'oxygène et
$R_1$ est en position 8 sur le cycle ainsi que leurs sels d'addition à des acides pharmaceutiquement acceptables.

**3.** La (*S*)-8-fluoro-4-méthyl-2-[4-(5-méthyl-1*H*-imidazol-4-yl)pipéridin-1-yl]-4,5-dihydroimidazo[1,5,4-*de*][1,4] benzoxazine ainsi que ses sels d'addition à des acides pharmaceutiquement acceptables.

**4.** La (*S*)-8-fluoro-4-méthyl-2-[4-(1*H*-imidazol-4-yl) pipéridin-1-y1]-4,5-dihydroimidazo[1,5,4-*de*][1,4] benzoxazine ainsi que ses sels d'addition à des acides pharmaceutiquement acceptables.

**5.** Procédé de préparation des composés de formule (I) selon la revendication 1, caractérisé en que l'on fait réagir un composé de formule (II)

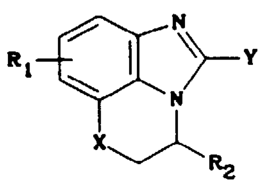

(II)

dans laquelle X, $R_1$ et $R_2$ sont définis comme dans la revendication 1 et Y représente un atome d'halogène, en particulier un atome de chlore,
avec un composé de formule (III)

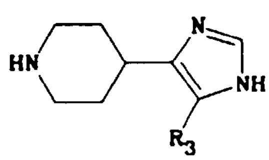

(III)

dans laquelle $R_3$ est défini comme dans la revendication 1.

**6.** Médicament caractérisé en ce qu'il contient un composé de formule (I) selon l'une quelconque des revendications 1 à 4.

**7.** Composition pharmaceutique caractérisée en ce qu'elle est constituée d'un composé de formule (I) selon l'une quelconque des revendications 1 à 4 en association avec des excipients appropriés.

Office européen des brevets

# RAPPORT DE RECHERCHE EUROPEENNE

Numero de la demande
EP 96 40 0452

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| D,A | EP-A-0 507 650 (SYNTHELABO)<br>* revendications 1,6,7 *<br>--- | 1,6,7 | C07D471/06<br>C07D498/06<br>A61K31/535<br>A61K31/435<br>//(C07D471/06, 235:00, 221:00), (C07D498/06, 265:00,235:00) |
| A | EP-A-0 591 026 (SYNTHELABO)<br>* revendications 1,5,6 *<br>--- | 1,6,7 | |
| P,X | EP-A-0 646 583 (SYNTHELABO)<br>* revendications 1,3,4 *<br>----- | 1,6,7 | |

DOMAINES TECHNIQUES RECHERCHES (Int.Cl.6)
C07D
A61K

Le présent rapport a été établi pour toutes les revendications

| Lieu de la recherche | Date d'achèvement de la recherche | Examinateur |
|---|---|---|
| LA HAYE | 6 Mai 1996 | Voyiazoglou, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)